# EUROPÄISCHE PATENTSCHRIFT

(11) **EP 0 785 986 B1**
(45) Veröffentlichungstag und Bekanntmachung des Hinweises auf die Patenterteilung: **06.03.2002**
(21) Anmeldenummer: 95935328.5
(22) Anmeldetag: 07.10.1995
(51) Int. Cl.: C12M 1/40, C12P 1/00

(54) **VERFAHREN ZUR KONTINUIERLICHEN ENZYMATISCHEN GEWINNUNG HYDROPHOBER PRODUKTE UND DAFÜR GEEIGNETE VORRICHTUNG**
PROCESS FOR THE CONTINUOUS ENZYMATIC EXTRACTION OF HYDROPHOBIC PRODUCTS AND DEVICE SUITABLE THEREFOR
PROCEDE D'EXTRACTION ENZYMATIQUE EN CONTINU DE PRODUITS HYDROPHOBES ET DISPOSITIF CORRESPONDANT

(30) Priorität: 11.10.1994 DE 4436149
(43) Veröffentlichungstag der Anmeldung: 30.07.1997
(73) Patentinhaber: FORSCHUNGSZENTRUM JÜLICH GMBH, 52425 Jülich (DE)
(72) Erfinder: KRUSE, Wolfgang, D-65719 Hofheim (DE); KRAGL, Udo, D-52428 Jülich (DE); WANDREY, Christian, D-52428 Jülich (DE)
(86) Internationale Anmeldenummer: DE9501399
(87) Internationale Veröffentlichungsnummer: WO9611256

(56) Entgegenhaltungen:
- EP-A- 0 273 679
- WO-A-87/02381
- WO-A-88/07075
- WO-A-88/07582
- US-A- 3 915 802

## Beschreibung

Die Erfindung bezieht sich auf ein Verfahren zur kontinuierlichen enzymkatalytischen Gewinnung hydrophober Produkte in wäßriger Lösung sowie auf die Verwendung einer dafür geeigneten Vorrichtung.

Biotransformationen im allgemeinen und enzymatische Verfahren im besonderen gewinnen zunehmend an Bedeutung für die Synthese von optisch aktiven Substanzen, aber auch für Bulkchemikalien, wobei von der im allgemeinen äußerst selektiven chemischen Wirksamkeit der im wäßrigen Milieu aktiven Enzyme Gebrauch gemacht wird.

Schwierigkeiten entstehen, wenn Substrate umgesetzt werden sollen und Produkte entstehen, die schlecht wasserlöslich sind. Zur Lösung dieses Problems existieren verschiedene Ansätze wie z.B. die Anwendung der Enzyme in auf einem festen Träger immobilisierter Form, der im organischen Lösungsmittel suspendiert wird, der Einsatz der Enzyme in homogener Lösung mit einem wassermischbaren Lösungsmittel oder der Einschluß von Enzymen in Reversmicellen.

Die direkte Gegenwart von organischen Lösungsmitteln (wassermischbar oder nicht wassermischbar) wird nur von wenigen Enzymen vertragen, die zur Klasse der Hydrolasen gehören, (z.B. WO 89/04784). Solche Enzyme werden dabei in der Regel zweidimensional in einer Membran immobilisiert eingesetzt (z.B. WO-A 88/07582; EP-A 0 273 679; WO-A 87 02381) oder beispielsweise durch Einschluß in ein Hydrogel immobilisiert (WO-A 88/07075). Unter dem Gesichtspunkt der besseren Katalysatorausnutzung in Bezug auf Aktivität und produktmengen-spezifischem Verbrauch sowie einer leichteren Handhabung wird ein homogen in Lösung verteilter Katalysator vensendet.

Da - wie bereits erwähnt - die Gegenwart größerer Mengen organischer Lösungsmittel im Reaktionsraum zur Desaktivierung bzw. Denaturierung der Enzyme führt, wird erfindungsgemäß auf organische Lösungsmittel im Reaktionsraum verzichtet und durch eine direkte kontinuierliche Einspeisung des umzusetzenden Substrats im Reaktionsraum eine Substratkonzentration entsprechend der maximalen Löslichkeit desselben aufrechterhalten und das sich bildende hydrophobe Produkt (zusammen mit Substratresten) aus der Reaktionsmischung über einen selektiven Trennvorgang entfernt (unter Anwendung einer entsprechend produktdurchlässigen Membran von ausreichender Fläche), während die wasserlöslichen Komponenten der Reaktionsmischung unverändert in Lösung bleiben. Die Produktentfernung kann beispielsweise mit einer Dialyse-Membran erfolgen (US-A 3/915 802). In EP-A 0 273 679 erfolgt die Produktabtrennung dadurch, daß der substrathaltige Zulauf an einer Membran mit immobilisiertem Enzym entlagströmt und das Produkt im Gegenstrom in einem Lösungsmittel, welches mit dem Lösungsmittel des Substratzulaufs nicht mischbar ist, entfernt wird.

Das erfindungsgemäße Verfahren der eingangs genannten Art ist mithin im wesentlichen dadurch gekennzeichnet daß man das Produkt aus der produkthaltigen Reaktionsmischung über eine produktdurchlässige, mikroporöse Membran in ein organisches Lösungsmittel extrahiert und die produkt-verarmte Reaktionsmischung zur Produktion rezykliert. Auch aus US-A 3/915 802 ist die Rückführung der Reaktionslösung bekannt, um eine kontinuierliche Produktentfernung zu ermöglichen.

Als "mikroporös" werden hier perforierte Membranen verstanden mit Porengrößen bis herab zu wenigen nm, insbesondere aber ≥ 10 nm. Die Obergrenze ist nicht kritisch. Voraussetzung ist, daß die Phasengrenze stabilisiert wird. So kann mit Porengrößen von 10 µm und darüber eine sichere Arbeitsweise erreicht werden. Die Membrandicke soll - insbesondere bei sehr feinporigen Membranen - für einen intensiven Austausch möglichst gering sein.

Vorzugsweise wird dabei mit einer mikroporösen hydrophoben Membran gearbeitet, speziell mit einem Hohlfasermembranbündel, wodurch große Membranflächen und geringe Flüssigkeitsschichtdicken realisiert werden können, die eine der Geschwindigkeit der enzymatischen Produktbildung angemessene Produktabtrennung zulassen. Der Einsatz von Hohlfasermembranbündeln in Enzym-Reaktoren ist auch aus EP-A 0 273 679 und WO-A 87/02381 bekannt.

Weitere Besonderheiten ergeben sich aus den Patentansprüchen und der nachfolgenden Beschreibung.

Zwar ist aus der DE 40 41 896 Cl ein Verfahren zur enzymatischen Synthese von organischen Verbindungen bekannt, bei dem die im Reaktionsraum gebildete "organische Verbindung" vom Enzym bzw. den Enzymen durch eine Lösungsdiffusionsmembran abgetrennt wird, die funktionelle Gruppen mit selektiver Wechselwirkung zur abzutrennenden Verbindung enthält. Dabei werden z.B. sulfonierte Polymermembranen vorgesehen und die organische Verbindung durch pervaporativen oder pertraktiven Austrag von der Membran abgeführt. Im letzteren Falle erfolgt die "Desorption" mit einer sogenannten Spülflüssigkeit, der z.B. zur Cyanhydrin-Abtrennung Methylenchlorid, Essigsäureanhydrid und alternativ Acethylchlorid zugesetzt werden.

Im Zusammenhang mit dieser Variante zu der seit langem bekannten Selektion von Komponenten mit Hilfe von Membranen wird auf die Nichtporosität derselben besonders hingewiesen, insbesondere unter Bezugnahme auf den Enzym-Membran-Reaktor, bei dem ein Reaktionsgemisch vom höhermolekularen Enzym getrennt wird.

Erfindungsgemäß erfolgt dem gegenüber eine selektive Produktabtrennung eines hydrophoben Produkts aus einer Reaktionsmischung durch eine Abwandlung der Flüssig/Flüssig-Extraktion, bei der eine an Mikroporen gebildete Grenzfläche- insbesondere im Gegenstrom - kontinuierlich erneuert wird.

Grundsätzlich ist die Erfindung durch Anwendung solcher Membranpatronen realisierbar, die in einen Produktionskreislauf eingesetzt werden, in dem die wäßrige Phase rezykliert. Bei einer solchen Ausgestaltung kommt das Enzym an der membranstabilisierten Grenzfläche mit dem organischen Lösungsmittel bei Verwendung mikroporöser Membranen in Kontakt, was gegebenenfalls zu einer Beeinträchtigung der Enzymaktivität führen kann.

Erfindungsgemäß kann durch direkte Einspeisung der reinen Edukte und Austrag der hydrophoben Produkte eine produktabgereicherte Reaktionsmischung mit darin gelösten Enzymen und Cofaktoren rezykliert werden, was zur Erhöhung der Zykluszahlen und damit zu einer Kostenreduzierung führt.

Besonders vorteilhaft werden erfindungsgemäß Hohlfaserbündel aus hydrophoben Materialien, wie gegebenenfalls fluorierten Kohlenwasserstoffpolymeren, wie insbesondere Polypropylen und Polyäthylen verwendet, deren relativ hohe Porosität und geringe Wandstärke bei relativ kleiner Porenöffnung für eine zweckmäßige Realisierung der Erfindung geeignet sind.

Es werden mikroporöse Membranen angewandt, die hydrophob oder auch hydrophil sein können, vorzugsweise aber hydrophob sein sollten.

Die organische Lösungsmittelphase kommt an der durch die Membran gebildeten Grenzfläche mit der wäßrigen Phase in Kontakt und würde (bei Verwendung einer hydrophoben Membran) in deren Raum übertreten, weshalb für eine relativ geringe positive Druckdifferenz zwischen wäßriger und organischer Phase gesorgt wird.

Nachfolgend wird die Erfindung anhand der beigefügten Zeichnungen sowie eines Durchführungsbeispiels näher erläutert; es zeigen schematisch:
- Figur 1:: ein Reaktionsschema;
- Figur 2:: ein Extraktionsmodul;
- Figur 3:: ein Extraktionsmodul eingebunden in eine erfindungsgemäße Produktionsanlage
- Figur 4:: eine Anordnung gemäß Fig. 3 gekoppelt mit einer kontinuierlichen Extraktionsmittelrektifikation; und
- Figur 5:: eine erfindungsgemäße Produktionsanlage mit einem dem Extraktionsmodul vorgeschalteten Enzymmembranreaktor.

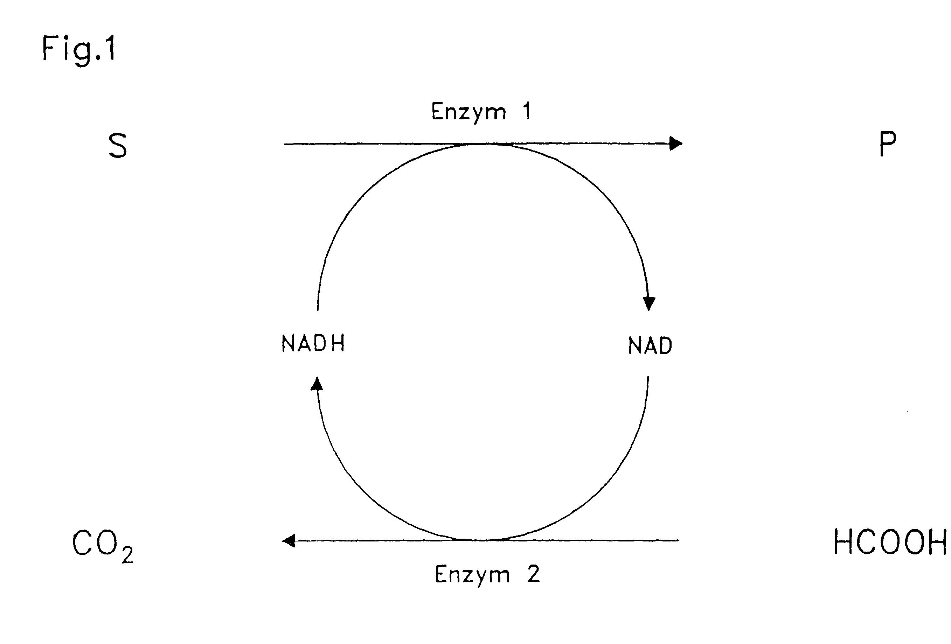

In Figur 1 ist eine typische Reaktion gezeigt, die in einer erfindungsgemäßen Anlage durchgeführt werden kann. Ein hydrophobes Substrat S wird in einer durch ein Enzym 1 katalysierten Reaktion zu einem hydrophoben Produkt P umgesetzt. Dazu wird ein Cofaktor, hier NADH, benötigt, der zu NAD abreagiert und in einer zweiten, durch ein Enzym 2 katalysierten Reaktion (Oxidation von Ameisensäure zu CO₂) regeneriert wird.

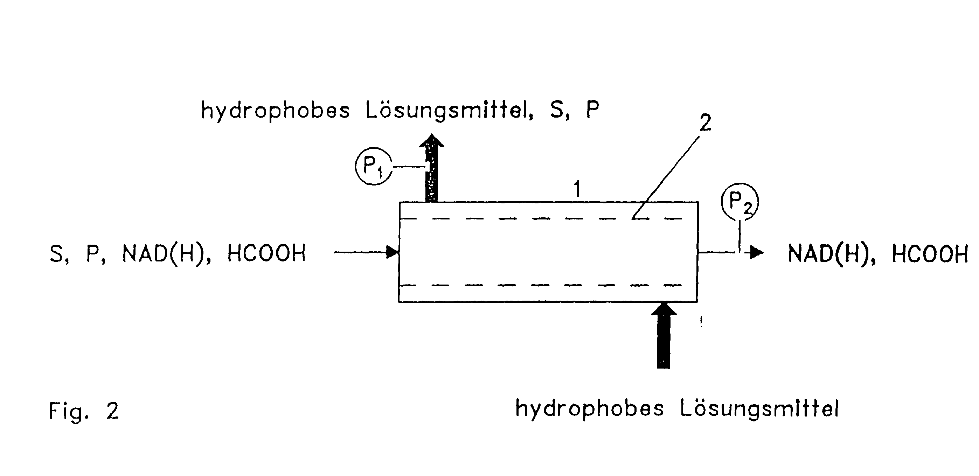

In Figur 2 wird ein Extraktionsmodul (1) gezeigt, das Bestandteil einer erfindungsgemäßen Anlage sein kann. Hier wird aus einer typischen wäßrigen Produktlösung mit Puffer, nicht umgesetztem Substrat S, durch enzymkatatysierte Reaktion entstandenem Produkt P, Cofaktor in reduzierter (NADH) und oxidierter Form (NAD) und Cosubstrat (in diesem Falle Ameisensäure (HCOOH)) über eine poröse Membran (2) mittels eines hydrophoben Lösungsmittels im Gegenstrom selektiv Substrat S und Produkt P extrahiert. Der Puffer, der Cofaktor (NAD(H)) und das Cosubstrat (HCOOH) verbleiben aufgrund ihrer Hydrophilie in der wäßrigen Phase.

Die Membran (2) dient zur Stabilisierung der Phasengrenzfläche zwischen hydrophober und wäßriger Phase. Die Auslegung der Membran (2) als Hohlfaserbündel fährt zu großen Phasengrenzflächen, was einen schnellen Stoffübergang befördert. Um, im Falle einer hydrophoben, porösen Membran das Penetrieren des hydrophoben Lösungsmittels in die wäßrige Phase zu verhindern, muß auf der Seite des wäßrigen Mediums ein Überdruck (P₂ > P₁) aufgeprägt werden. Desgleichen muß bei Nutzung einer *hydrophilen*, porösen Membran ein Überdruck (P₁ ≥ P₂) auf der Seite des hydrophoben Lösungsmittels aufgeprägt werden, um das Austreten der wäßrigen Reaktorlösung in die hydrophobe Phase zu verhindern.

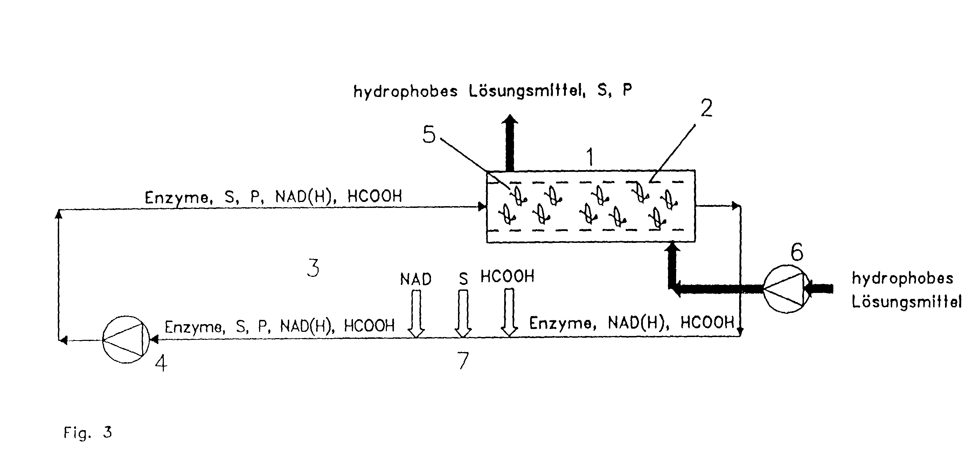

Die in Figur 3 skizzierte erfindungsgemäße Anlage bindet das in Figur 1 dargestellte Extraktionsmodul (1) in einen Reaktorkreislauf (3) ein. Die Reaktionslösung wir durch die Umlaufpumpe (4) im Reaktorkreislauf (3) geführt. Neben den Enzymen (5) enthält die Reaktorlösung vor dem Extraktionsmodul (1) die Cofaktoren NAD und NADH, das Cosubstrat HCOOH, nicht umgesetztes Substrat S und durch enzym-katalysierte Reaktion entstandenes Produkt P. Letztere werden im Extraktionsmodul (1) in das hydrophobe Lösungsmittel extrahiert, welches im Gegenstrom zur wäßrigen Phase mit der Extraktionsmittelpumpe (6) in das Extraktionsmodul gepumpt wird. Dem das Extraktionsmodul vorlassenden wäßrigen Strom mit den verbliebenen hydrophilen Bestandteilen (Enzyme, NAD(H), HCOOH) wird durch Nachdosierung bei (7) wieder Substrat S zugesetzt. Ebenfalls wird durch die Nachdosierung bei 7 durch Reaktion verbrauchtes Cosubstrat HCOOH und durch Deaktivierung verlorener Cofaktor NAD ersetzt.

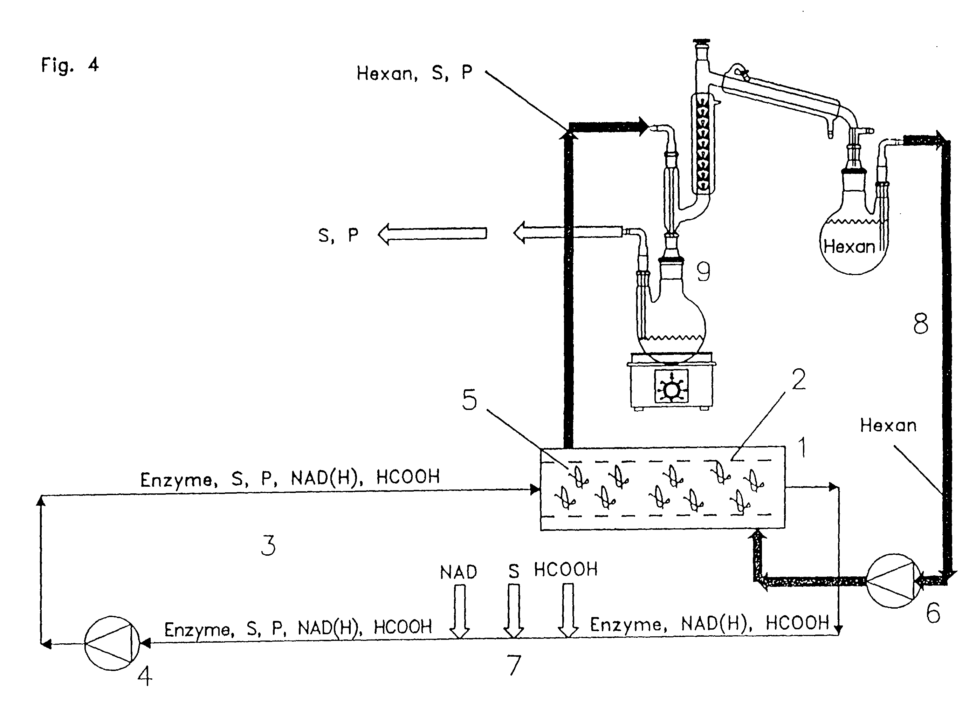

Die in Figur 4 gezeigte Anordnung entspricht z. T. Figur 3 mit Extraktionsmodul (1) und Reaktorkreislauf (3); sie ist jedoch durch einen Hexankreislauf (8) erweitert. In diesen Hexankreislauf (8) ist eine kontinuierliche Rektifikationskolonne (9) eingebunden, in der die extrahierten Substanzen (Substrat S, Produkt P) vom Extraktionsmittel Hexan getrennt werden.

Rektifiziertes Hexan wird durch die Extraktionsmittelpumpe (6) wieder dem Extraktionsmodul (1) zugeführt. Somit wird die effektive zur Extraktion benötigte Lösungsmittelmenge verringert.

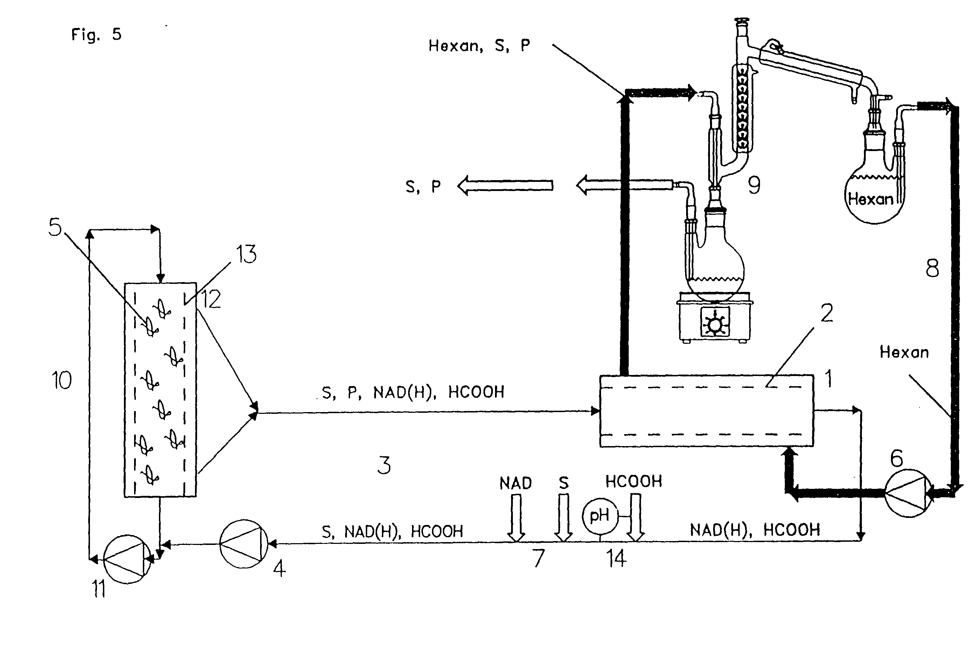

Die in Figur 5 dargestellte erfindungsgemäße Anordnung besteht aus einem Reaktorkreislauf (3), in dem durch die Reaktorkreislaufpumpe (4) eine Substratlösung bestehen aus Substrat S, Cofaktoren NAD(H) und Cosubstrat HCOOH in den Enzymmembranreaktor (10) gepumpt wird. Der Enzymmembranreaktor (10) ist als Kreislauf ausgelegt, der aus der Enzymkreislaufpumpe (11) und dem Ultrafiltrationshohlfasermodul (12) besteht. In diesem Kreislauf sind die Enzyme (5) hinter einer hydrophilen Ultrafiltrationsmembran (13) immobilisiert.

Aus der auf der Filtratseite des Ultrafiltrationshohlfasermoduls (12) den Enzymmembranreaktor (10) verlassenden enzymfreien Produktlösung wird im Extraktionsmodul (1) das nicht umgesetzte Substrat S und das entstandene Produkt P in einen kontinuierlichen Hexanstrom extrahiert. Der Substrat S und Produkt P enthaltende Hexanstrom wird in einer kontinuierlichen Rektifikationskolonne (9) in ein Substrat S /Produkt P Gemisch und Hexan getrennt. Das Hexan wird über den Hexankreislauf (8) wieder dem Extraktionsmodul (1) zugeführt.

Die durch die Extraktion an Produkt P verarmte, wäßrige Lösung kann durch die Nachdosierung von Substrat S und Cofaktor NAD bei (7) und durch ph-geregelte Nachdosierung von Cosubstrat HCOOH bei (14) wieder als Substratlösung dem Enzymmembranreaktor (10) zugeführt werden.

Die in Figur 5 skizzierte Anordnung wurde zur Produktion von (S)-1-Phenyl-2-Propanol verwendet. Die Prozeßbedingungen sind im nachfolgenden Beispiel aufgeführt.

### Beispiel 1

In einen 50 ml-Enzymmembranreaktor (EMR) mit einem Ultrafiltrationshohlfasermodul (Amicon hollow fibre, Typ H1P10-43, cut off: 10000 Da) mit zugeordneter Kreislaufpumpe wurden die Enzyme:
Alkoholdehydrogenase aus *Rhodococcus erythropolis:* 0,9 U/ml EMR-Vol.
Formiatdehydrogenase aus *candida boidinii :* 1,9 U/ml EMR-Vol.
gegeben und als Reaktionsmischung eine wäßrige Lösung von pH 6,7 mit

| | |
|---|---|
| Phenylaceton | 9 mM |
| NAD | 0,124 mM |
| Natriumformiat | 50 mM |
| Ameisensäure | 14,15 mM |
| di-Kaliumhydrogenphosphat | 60 mM |

zugeführt.

Bei einer mittleren Verweilzeit von 0,33 h im EMR (Gesamtreaktorumlaufstrom: 150 ml/h) und einer Reaktionstemperatur von 20 °C betrug der Umsatz bezüglich des Substrates Phenylaceton 73%.

Die den Enzymmembranreaktor verlassende Produktlösung wurde kontinuierlich einem mit einer hydrophoben, mikroporösen Polypropylenmembran (Membranfläche: 0,23 m²; effektive Porengröße: 0,05 µm) ausgestatteten Flüssig/Flüssig-Hohlfaserextraktionsmodul (Liqui-Cel^{R} Phasenkontakt-Labormodul 5PCM - 106 der Hoechst Celanes Corp.) zugeführt. Hier wurde durch einen kontinuierlichen Hexangegenstrom (1000 ml/h) aus der Produktlösung kontinuierlich (S)-1-Phenyl-2-Propanol und das nicht umgesetzte Phenylaceton extrahiert. Die produkthaltige Hexanlösung wurde einer kontinuierlichen Rektifikation zugeführt und rektifiziertes Hexan erneut zum Extraktionsmodul gepumpt. Das Gesamtvolumen der Hexanphase betrug 800 ml.

Das Penetrieren von Hexan durch die hydrophobe Membran wurde durch das Aufprägen eines Überdruckes von 0,5 bar auf der wäßrigen Seite verhindert.

Die das Extraktionsmodul verlassende produktfreie wäßrige Lösung wurde mittels kontinuierlicher Nachdosierung von Phenylaceton (1,35 mmol/h), ph-geregelter Nachdosierung von Ameisensäure und Ersetzen des durch Deaktivierung verlorenen Cofaktors (0,724 µmol/h) wieder als Reaktionslösung verwendet und erneut dem Enzymmembranreaktor zugeführt.

Das Gesamtvolumen der wäßrigen Phase betrug 450 ml.

Die Raum/Zeit-Ausbeute betrug bezogen auf das EMR-Volumen 64,3 g/(¹EMR^{*d}).

Die Wechselzahl des Cofaktors betrug 1361 molₚ/mol_{NAD} (73%*l, 35*1000/0, 724 µmolₚ/µmol_{NAD}).

Die Wechselzahl des Cofaktors im Vergleichsfall, d.h. ohne kontinuierliche Produktextraktion aus dem zum EMR zurückführenden Rezyklierungsstrom beträgt 53 molₚ/mol_{NAD}(73%*9, 0/0,124 mmolₚ/mmol_{NAD}).

Somit ergibt die erfindungsgemäße, extraktive Rezyklierung eine mehr als 25-fach bessere Ausnutzung des teuren Cofaktors.

Die erfindungsgemäße kontinuierliche Produktentnahme aus der Reaktionsflüssigkeit, die ansonsten insgesamt zum Enzymmembranreaktor zurückgeführt wird, macht die sonst notwendige stetige Einspeisung von Cofaktor in den EMR (im allgemeinen zusammen mit der Substratlösung) praktisch überflüssig; es müssen lediglich die bei wenigen Prozent der erforderlichen Menge liegenden (durch thermische Deaktivierung hervorgerufenen) Verluste ausgeglichen werden. Hydrophobe Membranen, insbesondere in Form von Hohlfaserbündeln, wie vorstehend erläutert, sind an sich seit gut 10 Jahren bekannt und erhältlich, ohne daß von ihrer außerordentlich nutzbringendem Wirkung für die enzymatische Gewinnung hydrophober Produkte Gebrauch gemacht worden wäre.

### Beispiel 2

Unter den gleichen Bedingungen wie in Beispiel 1 wurde 4-Phenyl-2-butanon zu (S)-4-Phenyl-2-butanol reduziert.

Durch kontinuierliche Substratzugabe wurde eine Zulaufkonzentration an 4-Phenyl-2-butanon in den EMR-Kreislauf von 12 mmol/L eingestellt. Um einen genügend hohen Umsatz zu erzielen, mußte die NAD(H)-Konzentration auf 1,2 mmol/L angehoben werden.

Bei einer Verweilzeit von 0,33 Std. im EMR betrug der mittlere Umsatz 80 % bei einer Raum Zeit-Ausbeute von 103,8 g/(l x d).

Entsprechend der Desaktivierung des Cofaktors wurde dieser mit 9,1 µmol/Std. nachdosiert.

Die Wechselzahl des Cofaktors betrug 158 molₚ/mol_{NAD}.

Die Wechselzahl des Cofaktors im Vergleichsfall, d.h. ohne Extraktion und Rückführung, beträgt 8 molₚ/mol_{NAD}. Die im Vergleich zum Beispiel 1 niedrigeren Werte für die Wechselzahl beruhen auf der notwendigen höheren Konzentration des Cofaktors von 1,2 mmol/L.

Dennoch ergibt sich auch hier durch die erfindungsgemäße extraktive Rezyklierung eine 20-fach bessere Ausnutzung des Cofaktors.

### Beispiel 3

Unter den gleichen Bedingungen wie in Beispiel 1 wurde 6-Methyl-5-hepten-2-on(S)-6-Methyl-5-hepten-2-ol ((S)-(+)-Sulcatol) reduziert. (S)-(+)-Sulcatol ist das männliche Pheromon der Borkenkäfer *Gnathotrichos sp.* und kann als Lockstoff in Fallen zur Schädlingsbekämpfung eingesetzt werden.

Durch kontinuierliche Substratzugabe wurde eine Zulaufkonzentration an 6-Methyl-5-hepten-2-on in den EMR-Kreislauf von 10 mmol/L eingestellt. Die stationäre NAD(H)-Konzentration wurde auf 0,2 mmol/L eingestellt.

Bei einer Verweilzeit von 0,33 Std. im EMR betrug der mittlere Umsatz 67 % bei einer Raum Zeit-Ausbeute von 60 g/(l x d).

Entsprechend der Desaktivierung des Cofaktors wurde dieser mit 1,5 µmol/Std. nachdosiert.

Die Wechselzahl des Cofaktors betrug 747 molₚ/mol_{NAD}.

Für den Vergleichsfall, d.h. ohne Extraktion des Produktes und Rückführung, beträgt die Wechselzahl 33 molₚ/mol_{NAD}.

Durch die Anwendung der erfindungsgemäßen extraktiven Rezyklierung wurde die Ausnutzung um den Faktor 23 verbessert.

## Patentansprüche

1. Verfahren zur kontinuierlichen enzym-katalysierten Gewinnung hydrophober Produkte in wäßriger Lösung,
**dadurch gekennzeichnet,**
**daß** die enzym-katalysierte Reaktion mit Reaktionspartnern durchgeführt wird, die homogen gelöst vorliegen und weiterhin dadurch, daß das Produkt aus der produkthaltigen Reaktionsmischung über eine produktdurchlässige mikroporöse Membran in ein organisches Lösungsmittel extrahiert wird und die produktverarmte Reaktionsmischung zur Produktion rezykliert wird.

2. Verfahren nach Anspruch 1,
**dadurch gekennzeichnet,**
**daß** eine hydrophobe Membran verwendet wird.

3. Verfahren nach Anspruch 1 oder 2,
**dadurch gekennzeichnet,**
**daß** eine cofaktor-abhängige enzymatische Umsetzung mit Cofaktor-Regenerierung durchgeführt wird.

4. Verfahren nach einem der vorangehenden Ansprüche,
**dadurch gekennzeichnet,**
**daß** man die produkthaltige Reaktionsmischung zur Abtrennung des Produkts durch ein Hohlfasermembranbündel schickt, das von außen von organischem Lösungsmittel umströmt wird.

5. Verfahren nach Anspruch 4,
**dadurch gekennzeichnet,**
**daß** das Hohlfasermembranbündel vom organischen Lösungsmittel im Gegenstrom zur Reaktionsmischung umströmt wird.

6. Verfahren nach einem der vorangehenden Ansprüche,
**dadurch gekennzeichnet,**
**daß** mit einer mikroporösen Membran mit einer effektiven Porengröße von 5 - 500 µm und mit einem Differenzdruck zwischen wäßriger Phase und organischem Lösungsmittel von 0,5-5 bar gearbeitet wird.

7. Verfahren nach einem der vorangehenden Ansprüche,
**gekennzeichnet durch**
die Produktion chiraler Alkohole, Amine, Cyanhydrine.

8. Verfahren nach einem der vorangehenden Ansprüche,
**gekennzeichnet durch**
einen Rezyklierungskreis für das organische Lösungsmittel, in den die Produktextraktion einbezogen ist.

9. Verfahren nach Anspruch 8,
**gekennzeichnet durch**
eine damit verbundene Produktisolierung und Rückführung von Substrat zur Produktion.

10. Verwendung einer Vorrichtung umfassend einen Produktionskreis, in den eine Extraktionspatrone mit produktdurchlässiger, mikroporöser Membran einbezogen ist, für die Durchführung eines Verfahrens nach den Ansprüchen 1 bis 8.

11. Verwendung einer Vorrichtung nach Anspruch 10,
**dadurch gekennzeichnet,**
**daß** für die Vorrichtung eine Extraktionspatrone in Form eines Hohlfasermembranbündels verwendet wird.

12. Verwendung einer Vorrichtung nach Anspruch 10 oder 11,
**dadurch gekennzeichnet,**
**daß** für die Vorrichtung Mittel zur Erzeugung eines Differenzdrucks zwischen wäßriger und organischer Phase in der Patrone eingesetzt werden.

13. Verwendung einer Vorrichtung nach einem der Ansprüche 10 - 12,
**dadurch gekennzeichnet,**
**daß** für die Vorrichtung eine Enzym-Membran-Reaktor-Anordnung für die enzymatische Umsetzung im Produktionskreis eingesetzt wird.

## Claims

1. A process for the continuous, enzyme-catalysed production of hydrophobic products in aqueous solution,
**characterised in that**
the enzyme-catalysed reaction is effected with reactants which are present in a homogeneously dissolved state and that, in addition, the product is extracted from the reaction mixture which contains the product into an organic solvent via a microporous membrane which is permeable to the product, and the reaction mixture which is depleted in product is recycled to the production stage.

2. A process according to claim 1,
**characterised in that**
a hydrophobic membrane is used.

3. A process according to claims 1 or 2,
**characterised in that**
a cofactor-dependent enzymatic reaction is carried out, with regeneration of the cofactor.

4. A process according to any one of the preceding claims,
**characterised in that**
the reaction mixture which contains the product is passed through a bundle of hollow fibre membranes, around the outside of which an organic solvent flows, in order to separate the product.

5. A process according to claim 4,
**characterised in that**
the organic solvent flows round the hollow fibre membrane bundle counter-current to the reaction mixture.

6. A process according to any one of the preceding claims,
**characterised in that**
a microporous membrane is employed which has an effective pore size of 5 - 500 µm, and that a differential pressure of 0.5 - 5 bar is employed between the aqueous phase and the organic solvent.

7. A process according to any one of the preceding claims,
**characterised by**
the production of chiral alcohols, amines or cyanohydrins.

8. A process according to any one of the preceding claims,
**characterised by**
a recycling circuit for the organic solvent, which circuit includes the extraction of the product.

9. A process according to claim 8,
**characterised by**
a product isolation stage and a stage comprising the recycling of the substrate to the production stage, which are combined with said process.

10. Use of an apparatus comprising a production circuit in which an extraction cartridge which comprises a microporous membrane permeable to the product is included for carrying out a process according to any one of claims 1 to 8.

11. Use of an apparatus according to claim 10,
**characterised in that**
an extraction cartridge in the form of a bundle of hollow fibre membranes is used for the apparatus.

12. Use of an apparatus according to claims 10 or 11,
**characterised in that**
means for producing a differential pressure between the aqueous and organic phase are employed in the cartridge for the apparatus.

13. Use of an apparatus according to any one of claims 10-12,
**characterised in that**
an enzyme-membrane-reactor arrangement for the enzymatic reaction in the production circuit is employed for the apparatus.

## Revendications

1. Procédé d'obtention en continu et catalysée par une enzyme de produits hydrophobes en solution aqueuse,
**caractérisé**
**en ce qu'**il consiste à effectuer la réaction catalysée par une enzyme sur des partenaires de réaction, qui se présentent à l'état dissous de manière homogène et en outre par le fait que le produit est extrait du mélange de réaction le contenant, par l'intermédiaire d'une membrane microporeuse perméable au produit, dans un solvant organique et le mélange de réaction appauvri en produit est recyclé à la production.

2. Procédé suivant la revendication 1, **caractérisé en ce qu'**il consiste à utiliser une membrane hydrophobe.

3. Procédé suivant la revendication 1 ou 2, **caractérisé en ce qu'**il consiste à effectuer la réaction enzymatique qui dépend d'un cofacteur, avec régénération du cofacteur.

4. Procédé suivant l'une des revendications précédentes, **caractérisé en ce que** l'on envoie le mélange de réaction contenant le produit, à la séparation du produit à travers un faisceau de membrane à fibre tubulaire, autour duquel passe à l'extérieur du solvant organique.

5. Procédé suivant la revendication 4, **caractérisé en ce que** le solvant organique passe autour du faisceau de membrane à fibre tubulaire, à contre-courant du mélange de réaction.

6. Procédé suivant l'une des revendications précédentes, **caractérisé en ce que** l'on opère avec une membrane microporeuse ayant une dimension effective de pore de 5 à 500 µm et en ayant une différence de pression, entre la phase aqueuse et le solvant organique, de 0,5 à 5 bars.

7. Procédé suivant l'une des revendications précédentes, **caractérisé par** la production d'alcools, d'amines et de cyanhydrine chiraux.

8. Procédé suivant l'une des revendications précédentes, **caractérisé par** un circuit de recyclage pour le solvant organique, dans lequel est inclus l'extraction du produit.

9. Procédé suivant la revendication 8, **caractérisé par** une isolation du produit qui y est reliée et par un recyclage du substrat à la production.

10. Utilisation d'un dispositif comprenant un circuit de production dans lequel est incluse une cartouche d'extraction ayant une membrane microporeuse perméable au produit, pour la mise en oeuvre d'un procédé suivant l'une des revendications 1 à 8.

11. Utilisation d'un dispositif suivant la revendication 10, **caractérisée en ce qu'**il est utilisé, pour le dispositif, une cartouche d'extraction sous la forme d'un faisceau de membrane à fibre tubulaire.

12. Utilisation d'un dispositif suivant la revendication 10 ou 11, **caractérisée en ce qu'**il est utilisé, pour le dispositif, des moyens de production d'une différence de pression entre la phase aqueuse et la phase organique dans la cartouche.

13. Utilisation d'un dispositif suivant l'une des revendications 10 à 12, **caractérisée en ce qu'**il est utilisé, pour le dispositif, un dispositif enzyme-membrane-réacteur pour la réaction enzymatique dans le circuit de production.
